# EUROPÄISCHE PATENTSCHRIFT

(11) **EP 0 814 824 B1**
(45) Veröffentlichungstag und Bekanntmachung des Hinweises auf die Patenterteilung: **09.05.2001**
(21) Anmeldenummer: 96907489.7
(22) Anmeldetag: 15.03.1996
(51) Int. Cl.: A61K 35/78, A61K 9/00

(54) **BRAUSEZUSAMMENSETZUNG MIT GINKGO-BILOBA TROCKENEXTRAKT**
EFFERVESCENT COMPOSITION WITH DRY EXTRACT OF GINKGO BILOBA
COMPOSITION EFFERVESCENTE A BASE D'EXTRAIT SEC DE GINKGO-BILOBA

(30) Priorität: 17.03.1995 DE 19509856
(43) Veröffentlichungstag der Anmeldung: 07.01.1998
(73) Patentinhaber: Dr. Willmar Schwabe GmbH & Co., 76227 Karlsruhe (DE)
(72) Erfinder: OSCHMANN, Rainer, D-76829 Landau (DE)
(74) Vertreter: Banzer, Hans-Jörg, Dipl.-Ing.
(86) Internationale Anmeldenummer: EP9601135
(87) Internationale Veröffentlichungsnummer: WO9629085

(56) Entgegenhaltungen:
- DATABASE WPI Section Ch, Week 9519 Derwent Publications Ltd., London, GB; Class D21, AN 95-144685 XP002007701 & JP,A,07 069 862 (KAO CORP.) , 14.März 1995

## Beschreibung

Durch eine Vielzahl von klinischen Versuchen ist belegt, daß der Wirkstoff aus Ginkgo-biloba Blättern, extrahiert mit Aceton-Wasser bei einer Reihe von Erkrankungen im Bereich peripherer und cerebraler Durchblutungsstörungen wirksam ist. Diese Befunde haben die Kommission E beim deutschen Bundesgesundheitsamt dazu veranlaßt, eine Monographie zu verabschieden (Bundesanzeiger Nr. 133 vom 19.7.1994) mit der Angabe folgender Indikationen:
- zur symptomatischen Behandlung von hirnorganisch bedingten Leistungsstörungen im Rahmen eines therapeutischen Gesamtkonzeptes bei dementiellen Syndromen mit der Leitsymptomatik: Gedächtnisstörungen, Konzentrationsstörungen, depressive Verstimmung, Schwindel, Ohrensausen, Kopfschmerzen;
- Verbesserung der schmerzfreien Gehstrecke bei peripherer, arterieller Verschlußkrankheit bei Stadium II nach Fontaine (Claudicatio intermittens) im Rahmen physikalisch-therapeutischer Maßnahmen, insbesondere Gehtraining;
- Schwindel, Tinnitus (Ohrgeräusche) vaskulärer und involutiver Genese.

Die Wirkung der Trockenextrakte aus Ginkgo-biloba Blättern kann durch eine therapeutisch wirksame Komponente oder durch die Kombination vieler wirksamer Komponenten hervorgerufen werden. Die wichtigsten therapeutisch wirksamen Komponenten sind Flavonoide, wie Ginkgoflavonglykoside und Terpenoide, wie Ginkgolide und Bilobalide. Dementsprechend werden die Arzneipräparate auf die genannten Komponenten standardisiert. Einer dieser Trockenextrakte, EGb 761, enthält etwa 24% Ginkgoflavonglykoside und etwa 6% Terpene; vgl. Jos Kleijnen, Paul Knipschild, The Lancet Nov. 7, 1992, Vol. 340, 1136. Weitere Trockenextrakte und Verfahren zu ihrer Herstellung und ihre Verwendung sind in den DE-Patentschriften 39 40 091 und 39 40 092 beschrieben.

Der Wirkstoff wird in der Regel in Form von Tabletten oder als Lösung appliziert, wobei nichtwäßrige Lösungsmittel, wie Ethanol oder Propylenglykol eingesetzt werden müssen. Rein wäßrige Lösungen sind nicht verfügbar, weil einerseits einzelne Komponenten eine sehr schlechte Löslichkeit in Wasser aufweisen, wie Ginkgolide von weniger als 0,02%. Andererseits sind einige Komponenten, wie Bilobalid in neutralem oder schwach basischem wäßrigen Milieu nicht stabil. Es treten in wäßrigen Lösungen sowohl Ausfällungen, als auch chemische Abbauprozesse von Extraktbestandteilen auf. Aus diesen Gründen liegt es nicht nahe, Brausezubereitungen zu fertigen. Zudem weist der Extrakt hygroskopische, klebende Eigenschaften auf, die die Verarbeitung zu Brausetabletten behindern.

Es besteht jedoch häufig der Bedarf an rein wäßrigen Zubereitungen eines Trockenextrakts aus Ginkgo-biloba Blättern. Der Erfindung liegt nun die Aufgabe zugrunde, eine Zubereitung von Trockenextrakten aus Ginkgo-biloba Blättern in Form einer Brausezusammensetzung zur Verfügung zu stellen, bei der nur Wasser als Lösungsmittel zum Auflösen der Brausezusammensetzung verwendet werden kann, bei der keine bemerkenswerten Abbauprozesse während der Anwendungsdauer stattfinden und mit einer Stabilität der nach dem Versetzen mit Wasser resultierenden Lösung von mindestens einer Stunde.

Diese Aufgabe wird durch Entwicklung einer Brausezusammensetzung gelöst, die einen Ginkgo-biloba Trockenextrakt sowie ein Brausegemisch umfaßt, das eine physiologisch verträgliche Säure oder deren Natriumsalz und ein physiologisch verträgliches Carbonat oder Hydrogencarbonat in einem solchen Mengenverhältnis enthält, so daß nach dem Versetzen der Brausezusammensetzung mit Wasser die resultierende Lösung einen pH-Wert von etwa 6 bis 8 hat und mindestens eine Stunde stabil ist.

Das Gewichtsverhältnis von Säure zu Carbonat oder Hydrogencarbonat beträgt vorzugsweise etwa 1:1 bis 1:3.

Durch Zusatz von Hilfsstoffen und durch die Festlegung der Bestandteile der Brausezusammensetzung und damit der Milieubedingungen der nach dem Versetzen mit Wasser resultierenden Lösung kann eine weitgehend klare Lösung erzielt werden, die über die Anwendungsdauer stabil ist. Nach dem Auflösen der Brausezusammensetzung in Wasser hat die resultierende Lösung einen pH-Wert von etwa 6 bis 8.

Unter dem Ausdruck "Brausezusammensetzung" werden hier z.B. Pulver, Granulate und Tabletten, vorzugsweise Tabletten verstanden.

Brausegemische enthalten physiologisch verträgliche Säuren und Carbonat bzw. Hydrogencarbonat zur Freisetzung von CO₂. Spezielle Beispiele für Carbonate und Hydrogencarbonate sind die Natriumsalze. Diese Brausegemische entwickeln beim Kontakt mit Wasser unter Zersetzung CO₂. Um die CO₂-Entwicklung zu optimieren, wird üblicherweise ein Überschuß an Säure zugesetzt. Dies bedeutet für die daraus resultierenden Lösungen leicht saure pH-Werte von 3 bis 5. Dieses Milieu ist jedoch für die Auflösung der Trockenextrakte aus Ginkgo-biloba Blättern ungünstig. Die Verwendung eines Unterschusses an Säure stellte sich überraschend als geeignet zur Herstellung der erfindungsgemäßen Lösungen heraus. Die CO₂-Entwicklung ist zwar unter diesen Bedingungen verringert, jedoch ist der resultierende pH-Wert von etwa 6 bis 8 geeignet zur Auflösung der Trockenextrakte.

Durch Zusatz geringer Mengen eines physiologisch verträglichen wasserlöslichen Tensids wird die Löslichkeit des Trockenextrakts zusätzlich positiv beeinflußt. Spezielle Beispiele für bevorzugt verwendete Tenside sind Polysorbate, d.h. nichtionische Tenside von Typ ethoxylierter Sorbitanester, Polyoxyethylenfettsäureester, ethoxylierter Glycerinester sowie Natriumdioctylsulfosuccinat. Das Tensid wird vorzugsweise in einer Menge von etwa 0,01 bis 0,5 Gew.-%, vorzugsweise 0,05 Gew.-% verwendet.

Ein akzeptabler Geschmack der resultierenden Lösung des sehr bitter schmeckenden Trockenextrakts kann durch Zusatz geeigneter Geschmackskorrigentien, wie Aromastoffen, Zucker bzw. Zuckeraustauschstoffen erreicht werden. Zucker oder Zuckeraustauschstoffe können in einer Menge von bis zu etwa 50 Gew.-% vorliegen. Zur Herstellung der erfindungsgemäßen Lösungen geeignete Geschmackskorrigentien sind künstliche oder natürliche Süßstoffe, vorzugsweise Saccharin-Na, Sorbit, Aspartam oder Mannit, vorzugsweise in einer Menge von etwa 0,5 bis 5 Gew-%, oder künstliche oder natürliche Aromastoffe, vorzugsweise Citronenaroma oder Grapefruitaroma und deren Gemische. Die Aromen werden vorzugsweise in einer Menge von etwa 0,5 bis 3 Gew.-%, vorzugsweise 1 Gew.-% verwendet.

Die Dosierung des Trockenextrakts pro Darreichungform, z.B. Tablette beträgt etwa 30 bis 240 mg.

Als Säuren geeignet zur Herstellung der erfindungsgemäßen Brausezusammensetzungen sind physiologisch verträgliche Säuren oder deren Gemische, vorzugsweise Citronensäure, Mononatriumcitrat und Weinsäure, insbesondere Citronensäure und Mononatriumcitrat.

Weiterhin werden wasserlösliche Tablettierhilfsstoffe, z.B. Füll- und Bindemittel, vorzugsweise Mannit, Lactose, Natriumsulfat, Polyvinylpyrrolidon oder deren Gemische und Hilfsstoffe, wie Antischaumemulsionen sowie Schmiermittel, wie Polyethylenglykole eingesetzt.

### Beispiel 1

Granulat bestehend aus:

| | |
|---|---|
| Trockenextrakt aus Ginkgo-biloba Blättern | 60 kg |
| Citronensäure | 160 kg |
| NaHCO₃ | 230 kg |
| Mannit | 250 kg |
| PEG 20000 | 33 kg |
| Saccharin-Na | 10 kg |
| Citronenaroma | 7 kg |

### Herstellung:

Trockenextrakt, Mannit und PEG 20000 werden gemahlen und gesiebt, die restlichen Bestandteile werden zugemischt. Das Gemisch wird zu Tabletten mit 13 mm Durchmesser und 800 mg verpreßt. Nach dem Auflösen innerhalb 3 min resultiert eine praktisch trübungsfreie Lösung. Die Wirkstoffe sind innerhalb einer Stunde stabil.

### Beispiel 2

Granulat bestehend aus:

| | |
|---|---|
| Trockenextrakt aus Ginkgo-biloba Blättern | 120 kg |
| Citronensäure | 320 kg |
| NaHCO₃ | 400 kg |
| Tween 80 | 0,6 kg |
| Mannit | 660 kg |
| PEG 20000 | 65,4 kg |
| Grapefruitaroma | 14 kg |
| Saccharin-Na | 20 kg |

### Herstellung:

Trockenextrakt und PEG 20000 werden gemischt und mit einer Lösung von Tween 80 (2% in Ethanol) versetzt. Die restlichen Bestandteile werden zugemischt. Das Gemisch wird zu Tabletten mit 18 mm Durchmesser und 160 mg verpreßt.

### Beispiel 3

Eine gemäß Beispiel 1 oder 2 hergestellte Tablette wird in 200 ml Wasser unter Bildung einer nahezu trübungsfreien Lösung gelöst. Die Lösung ist mindestens eine Stunde stabil, d.h. der Gehalt an Bilobalid beträgt mindestens 90% des Ausgangswertes.

### Beispiel 4

Granulat bestehend aus:

| | |
|---|---|
| Ginkgoextrakt | 80 kg |
| Natriumhydrogencitrat | 450 kg |
| Natriumcarbonat | 200 kg |
| Natriumhydrogencarbonat | 716 kg |
| Natriumsulfat | 420 kg |
| Lactose | 700 kg |
| Macrogol¹-Glycerolhydroxystearat | 5 kg |
| Simethicon² Antischaumemulsion | 15 kg |
| Aspartam Citronenaroma | 15 kg 55 kg |

| | |
|---|---|
| ¹ Polyethylenglykole | |
| ² mit Siliziumdioxid aktiviertes Polydimethylsiloxan | |

### Herstellung:

Aus dem Extrakt, Lactose, Macrogol-Glycerol-hydroxystearat und Simethicon wird durch Zusatz von Isopropanol ein Wirkstoffgranulat und aus den Citraten, Natriumsulfat und Natriumhydrogencarbonat ein Brausekörper gefertigt. Beide Granulate werden gemischt, mit den restlichen Hilfsstoffen versetzt und zu Brausetabletten mit 80 mg verpreßt.

## Patentansprüche

1. Brausezusammensetzung, umfassend einen Ginkgo-biloba Trockenextrakt und ein Brausegemisch aus einer physiologisch verträglichen Säure oder deren Natriumsalz und einem physiologisch verträglichen Carbonat oder Hydrogencarbonat in einem Gewichtsverhältnis von 1:1 bis 1:3, so daß nach dem Versetzen mit Wasser die resultierende Lösung einen pH-Wert von 6 bis 8 hat und mindestens eine Stunde stabil ist.

2. Brausezusammensetzung nach Anspruch 1, dadurch gekennzeichnet, daß die Säure Citronensäure oder Weinsäure oder deren Mononatriumsalz ist.

3. Brausezusammensetzung nach Anspruch 1, dadurch gekennzeichnet, daß das Carbonat oder Hydrogencarbonat das Natriumsalz ist.

4. Brausezusammensetzung nach einem der Ansprüche 1 bis 3, gekennzeichnet durch einen zusätzlichen Gehalt an einem physiologisch verträglichen wasserlöslichen Tensid.

5. Brausezusammensetzung nach Anspruch 4, dadurch gekennzeichnet, daß das Tensid ein Polysorbat oder Natriumdioctylsulfosuccinat ist.

6. Brausezusammensetzung nach einem der Ansprüche 1 bis 5, gekennzeichnet durch einen zusätzlichen Gehalt an einem Süßstoff.

7. Brausezusammensetzung nach einem der Ansprüche 1 bis 6, gekennzeichnet durch einen zusätzlichen Gehalt an einem Aromastoff.

## Claims

1. Effervescent composition comprising a dry extract of *ginkgo biloba* and an effervescent mixture of a physiologically acceptable acid or sodium salt thereof and a physiologically acceptable carbonate or hydrogen carbonate in a weight ratio of 1:1 to 1:3, so that the resulting solution after adding water has a pH value of 6 to 8 and is stable for at least one hour.

2. Effervescent composition according to claim 1, characterized in that the acid is citric acid or tartaric acid or the monosodium salt thereof.

3. Effervescent composition according to claim 1, characterized in that the carbonate or hydrogen carbonate is the sodium salt.

4. Effervescent composition according to any one of claims 1 to 3 characterized by additional content of a physiologically acceptable water soluble surfactant.

5. Effervescent composition according to claim 4, characterized in that the surfactant is a polysorbate or sodium dioctylsulfosuccinate.

6. Effervescent composition according to any one of claims 1 to 5, characterized by an additional content of a sweetening agent.

7. Effervescent composition according to any one of claims 1 to 6, characterized by an additional content of a flavouring agent.

## Revendications

1. Composition effervescente, comprenant un extrait sec de Ginkgo-biloba et un mélange effervescent d'un acide physiologiquement acceptable ou de son sel de sodium et d'un carbonate ou bicarbonate physiologiquement acceptable dans un rapport en poids de 1:1 à 1:3, de telle sorte que, après décomposition avec de l'eau, la solution résultante a une valeur de pH de 6 à 8 et est stable pendant au moins une heure.

2. Composition effervescente selon la revendication 1, caractérisée en ce que l'acide est l'acide citrique ou l'acide tartrique ou leur sel monosodique.

3. Composition effervescente selon la revendication 1, caractérisée en ce que le carbonate ou le bicarbonate est le sel de sodium.

4. Composition effervescente selon l'une des revendications 1 à 3, caractérisée par une teneur additionnelle en un agent de surface hydrosoluble physiologiquement acceptable.

5. Composition effervescente selon la revendication 4, caractérisée en ce que l'agent de surface est un polysorbate ou un dioctylsulfosuccinate de sodium.

6. Composition effervescente selon l'une des revendications 1 à 5, caractérisée par une teneur additionnelle en un édulcorant.

7. Composition effervescente selon l'une des revendications 1 à 6, caractérisée par une teneur additionnelle en un arôme.
